# EUROPEAN PATENT SPECIFICATION

(11) **EP 3 964 039 B1**
(45) Date of publication and mention of the grant of the patent: **03.07.2024**
(21) Application number: 20723122.6
(22) Date of filing: 01.05.2020
(51) Int. Cl.: H05K 1/18, A61M 15/00, H05K 3/30, H05K 3/32

(54) **INHALER COMPRISING AN ELECTRONIC MODULE**
INHALATOR MIT EINEM ELEKTRONISCHEN MODUL
INHALATEUR COMPRENANT UN MODULE ÉLECTRONIQUE

(30) Priority: 01.05.2019 GB 201906143
(43) Date of publication of application: 09.03.2022
(73) Proprietor: Norton (Waterford) Limited, Waterford (IE)
(72) Inventor: ROCHE, James, Enniscorthy, County Wexford (IE); CALDERON OLIVERAS, Enrique, 08172 Sant Cugat del Valles, Barcelona (ES)
(74) Representative: Kilburn & Strode LLP
(86) International application number: PCT/EP2020/062207
(87) International publication number: WO 2020/221925

(56) References cited:
- CN-U- 205 566 790
- JP-A- H03 159 221
- US-A1- 2005 019 654
- US-A1- 2011 110 063
- US-A1- 2015 061 867
- US-A1- 2016 166 768
- US-A1- 2016 235 916
- US-A1- 2018 140 788

## Description

This invention relates to an inhaler comprising an electronic module.

### BACKGROUND

A common problem faced in medical drug delivery devices such as respiratory drug delivery devices, regardless of the device used, is how to monitor patient adherence and compliance.

Adherence deals with the patient following the prescription label, for example taking the prescribed number of doses per day. If the prescription calls for two doses each day, and the patient is taking two doses a day, they are considered 100% adherent. If the patient is only taking one dose a day, they are only 50% adherent. In the latter case, the patient is not getting the treatment prescribed by their doctor.

Compliance, on the other hand, relates to how the patient uses their drug delivery device. If used in the manner recommended for effective treatment, they are 100% compliant. If not used properly however, they are less than 100% compliant. Use of an inhaler e.g. a dry powder inhaler involves inhaling in a particular way; for example the inhalation may need to be long enough and hard enough to entrain a full dose of medicament. For some patients, for example children and the elderly, meeting the requirements for full compliance may be difficult. However, failing to achieve 100% compliance can reduce the effectiveness of the prescribed medicament. When a doctor prescribes a medication, the efficacy of that treatment is totally dependent on the patient using their device properly and the proper number of times each day. If they fail to do so, the patient is likely to experience no improvement in their condition. Absent any means of verifying patient adherence/compliance, yet faced with a patient for whom no improvement can be seen, the doctor may have no choice but to prescribe a stronger dose or even a stronger medication. In some cases, this may put the patient at risk. This could be avoided if the doctor had some way of confirming that the patient was actually getting the medication prescribed.

Inhalers or puffers are examples of medical drug delivery devices used for delivering medication into the body via the lungs. They can be used, for example, in the treatment of asthma and chronic obstructive pulmonary disease (COPD). Types of inhalers include metered dose inhalers (MDIs), dry powder inhalers (DPIs), multi-dose powder inhalers (MDPIs) and nebulisers.

Electronic modules comprising electronic components - printed circuit boards (PCBs), processors, sensors (e.g. pressure sensors), batteries, transmitters, capacitors and the like - have been specially developed to be suitable for use with drug delivery devices in terms of, for example, compliance with regulations for medical devices and medical devices such as inhalers that work with powdered medicaments, and have been incorporated therein to develop how patient adherence and compliance are monitored.

The introduction of electronics into any drug delivery device may introduce certain technical challenges, such as durability, electro-mechanical integration, electrostatic effects, extractable volatiles and leachables, and drug delivery performance. It has been found that one challenge when incorporating electronics into a drug delivery device is ensuring the device is suitably durable, robust and resistant to mechanical shock; it is important that electronics incorporated into drug delivery devices do not become damaged and/or intermittently or permanently inoperable as a result of the device being dropped. A challenge specific to introducing electronics into powder-based drug delivery devices is ensuring that the electronics and associated components are compatible with the powders being used but still have the necessary properties to carry out their intended function. It is important to ensure the electronics being employed do not adversely interact with the powders in any way to degrade or alter the powders or have any electrostatic effects.

Drop tests have found that drug delivery devices such as inhalers comprising electronic components can become damaged, fail or experience intermittent power issues when the device is dropped and exposed to mechanical shock. Damage to internal components of drug delivery devices is undesirable, since not only can the damage lead to eventual failure of the device, but before failure occurs it can lead to the creation of particulate matter. Particulate matter loose inside a drug delivery device could be received (e.g. inhaled or ingested) by a patient along with the delivery of a drug. Any new materials incorporated into electronic modules must be considered carefully to ensure compatibility for the end use and that the necessary health and safety requirements are met e.g. they must be safe from a toxicology point of view.

US 2018/140788 A discloses a solution for inclusion of an electronic module in an inhaler. US 2011/110063 A1 discloses a robust consumer electronic device. US 2015/061867 A1 discloses devices, systems and methods for adherence monitoring and devices, systems and methods for monitoring use of consumable dispensers.

The present disclosure aims to alleviate at least to a certain extent one or more of the technical challenges posed by the introduction of electronics into a medical device, specifically an inhaler.

### SUMMARY

In a first aspect of the disclosure there is provided an inhaler and an electronic module for said inhaler, the electronic module comprising: a printed circuit board, and a damper configured to dampen energy transfer to and/or from a battery when a battery is connected to the electronic module and the electronic module is exposed to mechanical shock. The invention is defined in accordance with claim 1.

The electronic module may be exposed to mechanical shock when, for example, the module (or a medical device comprising the electronic module) collides with/ impacts a surface with a force, e.g. after having been dropped from a height. Impacts of sufficient force can induce vibrations throughout the electronic module (and throughout a medical device comprising the electronic module) and may cause deformations at the point of impact to some extent. On impact, kinetic energy of the electronic module (and of a medical device comprising the electronic module, if the module is part of a medical device) may be converted into heat and sound energy as a result of any vibrations and deformations.

Damping (i.e. reducing) energy transfer to and/or from the battery reduces vibrations of the battery that may result from a sudden impact. Battery movement and/or vibrations (often magnified by the flexible nature of a battery holder holding the battery inside the device) have been found to: damage printed circuit boards attached thereto, causing the printed circuit board to enter into an erroneous state and drain of the battery; cause damage and failure to fixings or heat stakes holding the printed circuit board to a part of the device; and cause battery holders holding the battery to the printed circuit board or other parts of the device to peel away from the printed circuit board. "Printed circuit board" as used herein relates to any form of printed circuit board and is meant to cover any printed circuit board assembly (PCBA). An electronic module in accordance with the first aspect mitigates against the aforementioned problems and is a notable departure from currently known electronic modules for medical devices such as inhalers. At least a part of the dampening may be provided through absorption of kinetic energy from the battery which may result when the electronic module is exposed to mechanical shock, thereby reducing energy transfer from the battery to the printed circuit board, or any other components of the electronic module.

Preferred and optional features of the first aspect will now be described.

The electronic module comprises a battery attached to the printed circuit board. The battery may be attached to the printed circuit board by one or more connectors. Preferably, the battery may be attached to the printed circuit board with two connectors. The one or more connectors may fixedly (non-detachably attached) attach the battery to the printed circuit board. One part of each connector may be attached to the battery, and another part of each connector may be attached to the printed circuit board. If more than one connector is provided, then preferably at least two connectors are attached to the battery on different or opposed sides thereof. The one or more connectors may be configured to provide electronic connection and/or mechanical connection between the battery and the printed circuit board. The one or more connectors may be tabs or wires, but preferably tabs.

Rigidly holding the battery to electrical terminals on the printed circuit board using the one or more connectors has been found to reduce power intermittency that can occur when the module is exposed to mechanical shock causing vibrations that temporarily separate the battery from the terminals. Connectors to hold a battery to a printed circuit board would generally be avoided since the connector-printed circuit board connection would be considered to deliver undesirable stress on the printed circuit board at the connection points due to the mass of the battery, leading to failure of components on the printed circuit board such as capacitors. However, it has been found that attaching the battery to the printed circuit board with one or more connectors (e.g. tabs) with the damper described herein advantageously reduces intermittency of power when the device is exposed to mechanical shock (as opposed to using a conventional battery holder/housing).

The damper may be attached to the battery and/or to one or more of the tabs.

The damper comprises a substrate configured to provide at least part of the damping. The substrate may be a layer. The substrate may be applied by adhesive to a surface of the battery connector. Optionally, the adhesive material is acrylic. Any other type of adhesive which is safe from a toxicology point of view may be used.

Whilst sticky materials such as adhesives sometimes are to be avoided in drug delivery devices - especially drug delivery devices for delivering powder based drugs - due to the possible adhesion of stray medicament (e.g. powder) thereto and/or components which may be given off during and after curing as extractable volatile components, it has been found that using adhesive as disclosed herein is a safe and effective way of attaching the substrate material to the battery connector.

The printed circuit board is attached to a cap or cover configured to be, preferably removably, attached to a surface of the inhaler. The cap may fully or partially cover (or enclose together with a housing of the inhaler) one or more of the other components of the electronic module when, preferably removably, attached to the surface. The cap may, in combination with the surface, fully or partially cover the other components of the electronic module when, preferably removably, attached to the surface, thereby reducing the risk of the other components of the electronic module directly contacting a surface if the inhaler is dropped.

The cap may be configured to enable mechanical and/or electronic connection between the electronic module and electronic components in a medical device such as pressure sensors, indicators (LEDs, alarms etc.), temperature sensors and the like.

The printed circuit board is attached to the cover or cap by one or more heat stakes, preferably two heat stakes. The one or more heat stakes may pass through the printed circuit board and into (or be integral with) the cover or cap in order to attach the printed circuit board thereto.

Fastening the printed circuit board to the cap using fasteners (e.g., screws, rivets, etc.) is likely to result in failure in drop tests, since the connection formed is rigid and unforgiving i.e. the operation and/or performance of the inhaler may be adversely impacted when the printed circuit board is attached to the cap only using fasteners. Using fasteners to fasten the printed circuit board to the cap may also increase manufacturing cost and/or manufacturing time. The heat stakes may be configured to partially deform when securing the printed circuit board to the cap, thereby connecting the printed circuit board to the cap or surface by creating an interference fit. The heat stakes are configured to have a degree of flexibility which allows them to dampen or completely absorb any mechanical energy from the PCB and/or the cap. Accordingly, the use of heat stakes improves durability of the electronics module. Two heat stakes are preferred since providing two gives a strong connection between the printed circuit board and cap, whilst allowing the heat stakes to flex and absorb any mechanical energy from the PCB and/or the cap. The use of heat stakes to fasten the printed circuit board to the cap may reduce manufacturing costs and/or manufacturing time.

The substrate may be configured so as not to cover the entire area of the surface of the battery to which is it applied, and preferably the substrate covers between 95 to 40% of the surface of the battery to which it is applied, more preferably 50 to 80 % and most preferably 80%. The substrate on the surface of the battery is preferably offset in a direction opposite to the one or more heat stakes. For example, the substrate may be applied to the battery so as to be offset in a direction away from being true/fully centred on the surface of the battery and away from the general direction of the heat stakes.

When developing the device disclosed herein the inventors found that, due to the compact nature of the device, the process of deforming the heat stakes by applied heat was degrading or even melting the applied substrate located near the heat stakes when the substrate was sized to the same size as the battery's surface. Degradation or melting of the substrate may adversely affect substrate performance, longevity, and result in unknown or unpredictable safety properties from, for example, a toxicology perspective (e.g. extractable/leachable properties). Making the substrate offset and smaller as described herein has been found to avoid this problem whilst effectively acting as a damper.

The battery connector(s) and/or the damper are configured to position the substrate at a surface, thereby enabling the substrate to provide compressive damping against the surface (e.g. to dampen energy transfer to and/or from the battery). The surface is a surface of the inhaler.

Compressive damping in this case may be where the substrate is pushed and compressed against the surface and absorbs mechanical energy transferred to and from the battery, thereby preventing the mechanical energy being transferred to other parts of the electronic module such as the printed circuit board, battery connector, and any sensors or indicators that may be provided.

There may be a distance between the substrate and the surface, said distance may be less than 5 mm once the electronic module is attached to the inhaler, preferably the distance is less than 2 mm. The substrate is loaded against the surface when the electronic module is attached to the inhaler i.e. the substrate of the damper is positioned so as to contact (and may optionally push against) the surface when the electronic module is attached to the inhaler. The substrate is loaded against the surface in compression.

Configuring the electronic module so that the substrate is loaded against the surface in this way applies additional assembly forces to the components of the electronic module. Whilst increased assembly forces are generally to be avoided - especially when trying to improve robustness and drop resistance of the electronic module - it has been found that loading the substrate against the surface in the way disclosed herein has no significant adverse effects but improves drop resistance; the action of the substrate against the surface reduces deflection of the components of the electronic module past their neutral point (the position they would be in when under no applied load) when the electronic module is exposed to mechanical shock. Since one or more heat stakes are present, the substrate loaded against the surface applies compressive forces to the heat stakes via the printed circuit board, and advantageously dampen deflection of the heat stakes past their neutral point when exposed to applied load (such as when the electronic module is exposed to mechanical shock). Accordingly, loading the substrate against the surface in the way described herein has been found to reduce likelihood of damage and/or failure as a result of the device being dropped.

The substrate is loaded against the surface to compress the substrate when the electronic module is attached to the inhaler. Preferably, the substrate (when attached to the inhaler) is compressed by 95 to 5 %, further preferably less than 50%, more preferably less than 25%, even more preferably less than 10%, where, for example, 95 % compressed means that the volume of the substrate under compression is 95 % that of its volume under no compression under atmospheric conditions.

The substrate may apply from 0 to 100 N of force (an assembly force) against the surface once the electronic module is attached to a medical device, preferably 0 to 20 N, more preferably 1 to 15 N, most preferably 2 to 10 N.

It has been found that if the force between the substrate and a surface is too high then there is a risk of plastically deforming the substrate, which is one reason why excessive assembly forces should be avoided. Material properties of the substrate (e.g. material strength) should be considered to avoid plastic deformation when the substrate is loaded against the surface. Plastic deformation of the substrate - especially when the substrate is a foam - has been found to adversely affect the damping ability thereof, since the substrate exhibits non-linear damping behaviour.

The substrate may have a 25% compression force deflection of between 0.1 KPa to 200 KPa, preferably 1 KPa to 100 KPa, more preferably 1 KPa to 80 KPa, even more preferably 5 KPa and 70 KPa.

Compression force deflection is a measure of strength of a cellular product (e.g. a foam material). 25% compression force deflection relates to the force required to compress the material to 25% of its thickness i.e. the amount of force that the material exerts at 25% compression. Compression force deflection of a material may be determined by any suitable method, for example using methods described in the ASTM D3574 (test c) standard or ISO 3386 standard.

The substrate may be from 0.1 to 10 mm thick, preferably from 0.1 to 3 or to 5 mm thick.

The substrate material may be a foam material. The substrate may comprise rubber, polyurethane, silicone, neoprene, polyethylene-vinyl acetate, or polyethylene such as low-density polyethylene. The substrate may comprise more than one layer (e.g. 2, 3, 4, 5, 7, 8, 9, 10 etc layers) formed of any combination of the aforementioned materials.

The electronic module may be configured to prevent communication of medicament (if the medical device comprises a medicament, e.g. a powdered medicament) held within the device with the printed circuit board and battery. The electronic module may further comprise a gasket, membrane or filter configured to prevent communication of the medicament with the printed circuit board and battery.

In a second aspect of the disclosure there is provided an electronic module for a medical device such as an inhaler, the electronic module comprising: a printed circuit board, and a battery that is mechanically and electronically connected to the printed circuit board by one or more connectors, the connectors configured to dampen energy transfer to and/or from the battery when the electronic module is exposed to mechanical shock.

The benefits of dampening energy transfer to and/or from the battery and the use of one or more connectors are discussed above in relation to the first aspect.

In a third aspect of the disclosure there is provided an electronic module for a medical device such as an inhaler, the device comprising a medicament, and the electronic module comprising: a printed circuit board and a battery, wherein the electronic module is configured to prevent communication of the medicament within the device with the printed circuit board and battery.

Isolating the electronic module from medicament in the medical device advantageously prevents medicament (e.g. powder) from communicating (i.e. physically contacting) and adversely interacting with the printed circuit board and battery.

The electronic module may further comprise a gasket, membrane or filter configured to prevent communication of the medicament with the printed circuit board and battery.

In a fourth aspect of the disclosure there is provided a medical device comprising an electronic module as in the first, second or third aspects. The medical device may be a portable medical device such as a drug delivery device, blood glucose monitor, blood oxygen monitor, blood pressure monitor, and injection pen. Preferably, the medical device is a drug delivery device such as an inhaler.

Any of the optional and preferred features described in connection with the first aspect may be considered optional and preferred features of the second, third and fourth aspects to equal effect.

In a fifth aspect of the disclosure there is provided a method of manufacturing an electronic module as described herein, the method comprising the steps of: providing a printed circuit board and providing a damper configured to dampen energy transfer to and/or from a battery when a battery is connected to the electronic module and the electronic module is exposed to mechanical shock.

In a sixth aspect of the disclosure there is provided a method of manufacturing an electronic module as described herein for a medical device such as an inhaler, the device comprising a medicament, the method comprising the steps of: providing a printed circuit board and a battery and configuring the electronics module to prevent communication of the medicament with the printed circuit board and the battery.

In a seventh aspect of the disclosure there is provided a method of manufacturing an electronic module as described herein for a medical device such as an inhaler, the method comprising the steps of providing a printed circuit board and a battery that is mechanically and electronically connected to the electronic module by one or more connectors, the connectors configured to dampen energy transfer to and/or from the battery when the electronic module is exposed to mechanical shock.

Any of the optional and preferred features described in connection with the first aspect may be considered optional and preferred features of the fifth, sixth and seventh aspects to equal effect.

### BRIEF DESCRIPTION OF DRAWINGS

The above and other aspects of the invention will now be described, by way of example only, with reference to the accompanying drawings, in which:
**FIG. 1** shows a partially-exploded view of an example inhaler with an electronics module;
**FIG. 2** shows a partially-exploded view of the example electronics module from a bottom-up perspective of the components;
**FIG. 3** shows a partially-exploded view of the example electronics module from a top-down perspective of the components;
**FIG. 4** shows a cross-section view of the example inhaler with the example electronics module attached; and
**FIG. 5** shows a view of the example electronic module comprising a damper offset from the heat stakes.

### DETAILED DESCRIPTION

A detailed description of an example electronics module for use in a respiratory drug delivery system shall now be given with respect to use in an inhaler device. It should be understood that use of the example electronics module described herein is not limited only to inhalers, and may be used, for example, in any portable medical device (which includes respiratory drug delivery systems) with similar or equal effect. A non-exhaustive list of portable medical devices includes inhalers, blood glucose monitors, blood oxygen monitors, blood pressure monitors, injection pens and the like.

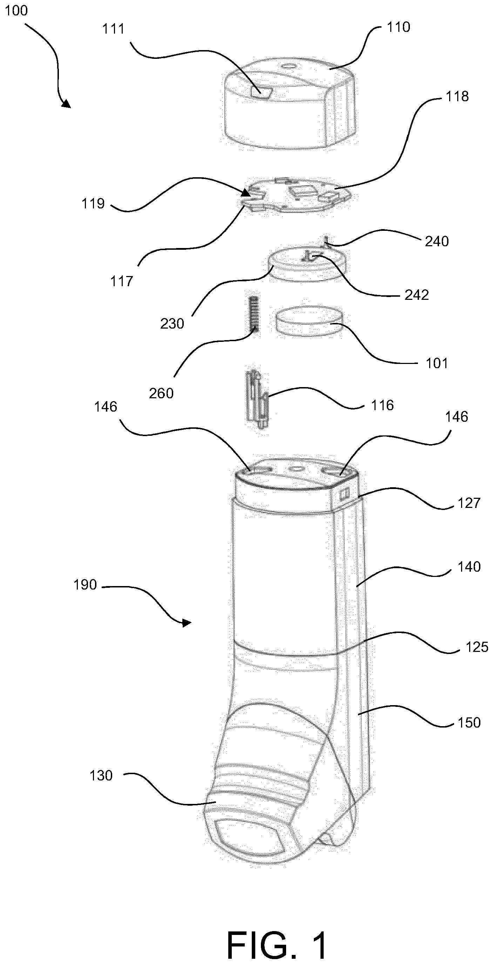

FIG. 1 shows a partially-exploded view of an inhaler 100. A housing 190 comprises an upper housing 140 which may interface with a lower housing 150. The upper housing 140 and the lower housing 150 may be removably or permanently attached to one another, thereby forming a seal 125. The inhaler 100 may also include an electronics module 105. The electronics module 105 may have a cap 110 (e.g., an electronics module cap) that interfaces with the upper housing 140. The cap 110 and the upper housing 140 may be removably or permanently attached to one another, thereby forming a seal at point 127. When the cap 110 is attached to the upper housing 140 a side wall 221, a top inner surface 220 and the top of upper housing 140 define an air space. This air space may be configured so that any drug/medicament to be delivered comprised within the inhaler 100 cannot enter (i.e. no fluid or solid particulate communication), although this requirement is not essential.

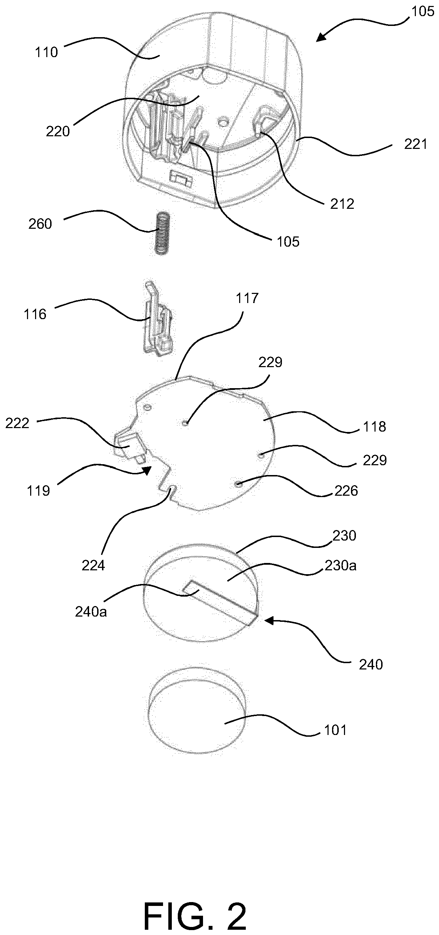

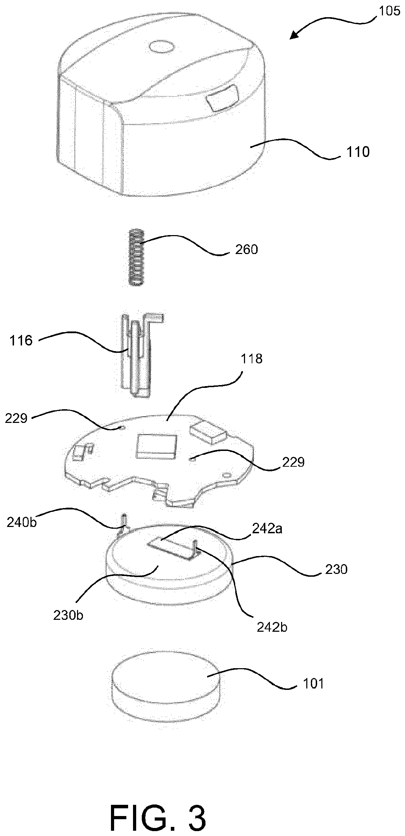

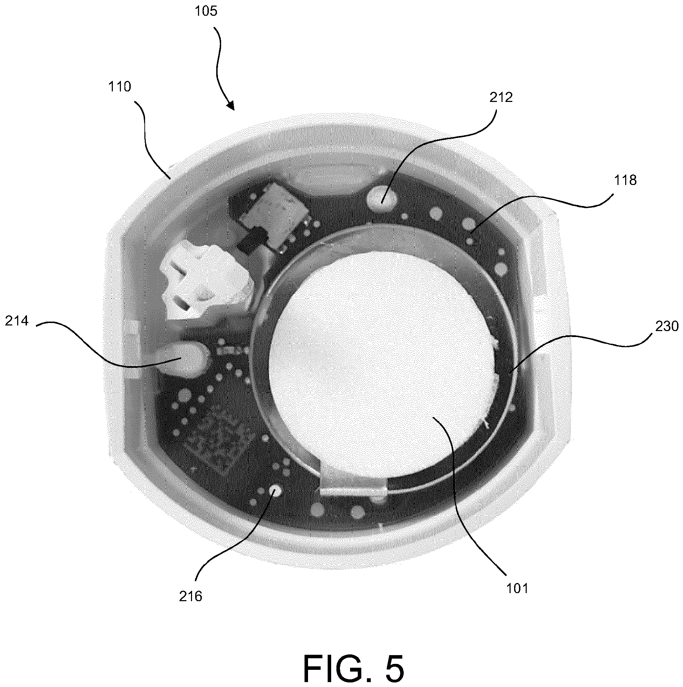

The cap 110 of the electronics module 105 may house a printed circuit board (PCB) 118, which may have an edge 117 that defines a notch 119. The PCB 118 may be attached to the cap 110 via a plurality of heat stakes 212, 214, as further described herein as shown in FIGs 2 and 5. The heat stakes 212, 214 may be configured to retain the PCB 118 within the cap 110. A slider 116 is housed substantially in the cap 110 and may be configured to activate the PCB 118 based on operation of a mouthpiece cover 130. For example, the slider 116 may move axially within the cap 110 to activate a switch 222 (shown in FIGs. 2 and 3) on the PCB 118 when the mouthpiece cover 130 is opened to expose the mouthpiece.

When the slider 116 is slidably mounted within the cap 110, a first (e.g., upper) portion of the slider 116 may protrude through the notch 119. A second (lower) portion of the slider 116 may protrude through one of the orifices 146 and extend into the upper housing 140. More than one orifice 146 may be provided to permit the upper housing 140 and/or the cap 110 to be rotated axially 180 degrees without affecting the manner in which they are attached to one another. As discussed further herein, a slider spring (e.g. the slider spring 260 shown in FIGs 1, 2, and 3) within the electronics module 105 may bias the slider 116 in a downward direction, i.e., push the slider towards the lower housing 150. As such, the slider spring may cause the end of the slider 116 within the upper housing 140 to maintain contact with, and continually rest against, a top surface of a yoke inside upper housing 140 (not shown in the FIGs). Thus, the slider 116 may move axially with the yoke along the same axis when the mouthpiece cover 130 is moved between the open and closed positions. The slider may transfer movement of an inhaler's yoke to the electronics module's switch 222. The movement of the inhaler's yoke may be associated with typical inhaler operation, for example the yoke may move in connection with the opening and closing of the inhaler's mouthpiece cover 130. As such, the slider spring may cause the end of the slider 116 within the upper housing 140 to maintain contact with, and continually rest against, a top surface of a yoke inside the upper housing 140 when the mouthpiece cover 130 is in the closed position and may cause the slider to decouple from the yoke when the mouthpiece cover 130 is in the open position. Here, the slider may effectively integrate the electronics module into an operation that is familiar to the user, improving the overall electromechanical integration of the inhaler. That is, activation of the electronics module may be transparent to the user as the user operates the inhaler. Cap 110 may comprise a substantially transparent section 111 configured to be positioned in the vicinity of an LED integrated with the electronics module, whereby the LED may be used to indicate inhaler operation to the user, e.g. the LED may be used to indicate activation of the electronics module due to the cover 130 being in an open position or when one or more doses of medicament have been administered from the inhaler.

The heat stakes 212 and 214 which fasten the PCB 118 to the cap 110 may be integrally formed with cap 110. FIG. 2 shows two integrally formed heat stakes 212 and 214, however, cap 110 is not limited to only comprising two heat stakes and may comprise more, e.g., 3, 4, 5, 6, 7, 8, 9, 10 etc. heat stakes. The heat stakes 212, 214 may protrude or extend from the top inner surface 220 of the cap 110. Cap 110 may comprise two types of heat stake. Heat stake 212 may have a circular cross sectional shape, but is not limited in this way and may have any other cross section, e.g., square, triangular etc... Heat stakes 212 may have a diameter that is smaller than a standard heat stake diameter. That is, the diameter of the heat stake 212 may be selected such that the inhaler 100 will successfully pass the drop test without taking up too much space on the PCB 118. Preferably, the heat stake 212 may have a diameter less than 1.4 mm. The PCB 118 may have a plurality of openings 224 and 226, as shown in FIGs 2 and 3. One or more of the openings (e.g., the opening 226) may correspond to the heat stake 212 such that the heat stake 212 may be adapted to protrude through the PCB 118 via the opening 226 when the PCB 118 is mounted within the cap 110.

Heat stake 214 may have a non-circular cross-section, for example, such as a rib- shaped cross-section. The notch 224 may correspond to the location of the stake 214, for example. The PCB 118 may define the notch 224 such that the stake 214 may be adapted to protrude through the PCB 118 via the notch 224 when the PCB 118 is mounted within the cap 110. Each of the heat stakes 212 and 214 may define a distal end that is opposite from the top inner surface 220 of the cap 110. The distal end of each of the heat stakes 212 and 214 may be configured to be partially deformed when heated to a predetermined temperature. When partially deformed, the distal end of the heat stakes 212 and 214 may take a substantially dome or semi spherical (e.g. ½ spherical) form. The partially deformed heat stakes 212 and 214 may secure the PCB 118 to the cap 110 and may be loaded in tension when doing so. An additional heat stake 216 (as shown in FIG. 5) may be provided. Heat stake 216 may be integrated with cap 110, protrude through the PCB 118 via an opening on the PCB when the PCB 118 is mounted within the cap 110, but may not be deformed as described above in connection with heat stakes 212 and 214.

The PCB 118 may further include a processor and a transmitter (not shown in the FIGs). The electronics module 105 may be installed on the upper housing 140 of the inhaler 100 towards the end of manufacture of the inhaler (e.g., following equilibration of the inhaler). Installing the electronics module 105 on the upper housing 140 towards the end of the manufacture of the inhaler 100 may be advantageous since equilibration of the inhaler 100 may damage the sensitive electronics on the PCB 118. Equilibration may involve filing the inhaler 100 with a medicament and storing the inhaler 100 at a predefined temperature and humidity for duration of time (e.g., four weeks) before final packing of the inhaler 100. Installing the electronics module 105 on the upper housing 140 of inhaler 100 avoids assembling electronic components in sterilised clean filling areas of the manufacturing facility.

A battery 230 (e.g., such as a coin cell) may be attached to the PCB 118 by way of tabs 240, 242 such that the battery 230 maintains contact with the PCB 118. Each of the tabs 240, 242 may comprise a first portion 240a, 242a configured to be attachable to the battery 230 and a second portion 240b, 242b configured to be attachable to the PCB 118, thereby attaching the PCB 118 to the battery 230. The first portion 240a comprises a substantially planar rectangular surface (tabs/strip portion) contiguous with one side 230a of the battery 230, and the first portion 242a comprises a substantially planar rectangular surface contiguous with another side 230b of the battery 230. Tab 240 may be attached to side 230a of battery 230 which is the opposite to side 230b of battery 230 to which tab 242 is attached. The second portion 240b, 242b is substantially perpendicular to the first portion 240a, 240b and comprises a planar rectangular surface (tabs/strip portion) which may be of reduced dimensions - preferably reduced width as shown in FIGs 2 and 3 - with respect to the first portion, but this is not essential. The PCB 118 may have a plurality of openings 229 as shown in FIGs. 2 and 3. The plurality of openings 229 may correspond to the second portion 240b, 242b such that the second portion 240b, 242b may be adapted to protrude through the PCB 118 via the openings 229 when the battery 230 is brought next to the PCB 118. For example, the tabs 240b, 242b may extend through openings 229 defined by the PCB 118 when the battery 230 is brought next to the PCB 118. The tabs 240b, 242b may be compliant such that the tabs deflect and engage the openings 229 such that the battery 230 is removably attached to the PCB 118. The tabs 240 and 242 may be configured such that an electrical connection may be formed between the PCB 118 and the battery 230 in addition to a mechanical connection, wherein the tabs provide an electrical connection between terminals on the battery 230 and electrical contacts on the PCB 118.

The first portion 240a, 242a and second portion 240b, 242b may be attached to the surface of the battery 230 by any suitable means, e.g., spot welding or adhesive. Once the second portion 240b, 242b has protruded through the openings 229 then the protruded portion may be bent, welded or soldered in place so as to fix the tabs 240, 242 (and therefore the battery 230) to the PCB 118. The battery 230 may alternatively be attached by one tab or more than two tabs in a similar way as shown for tabs 240, 242 in FIGs 2 and 3, for example there may be two, three, four, five etc. tabs 240, 242 attached to either side 230a, 230b of the battery with a corresponding number of openings 229 to receive them on the PCB 118.

One or more components of the PCB 118 may be selectively activated based on a position of the mouthpiece cover 130. For example, activation of the switch 222 (e.g., or activation of some other switching means, such as an optical sensor, an accelerometer, or a Hall effect sensor) may wake a processor and/or transmitter from an off state (or a power-conserving sleep mode) to an on state (or an active mode). Conversely, deactivation of the switch 222 may transition the processor and/or transmitter from the on state (or active mode) to an off state or a lower power mode.

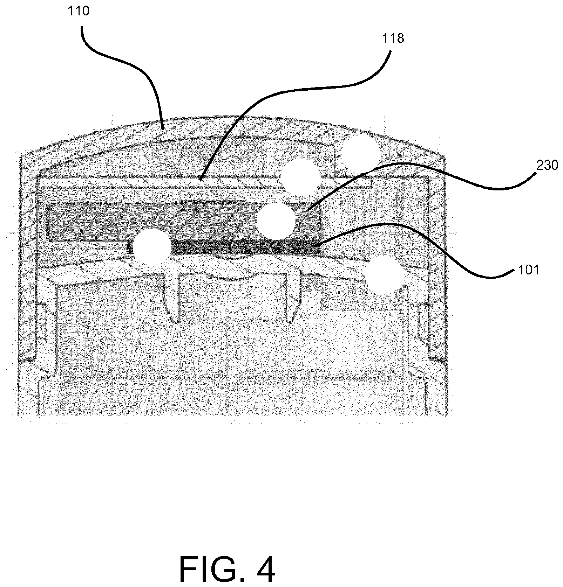

A damper 101 may be attached to the battery 230 (shown in FIGs 1 to 5) for dampening energy transfer to and/or from the battery 230 when attached to the PCB 118 and when the electronic module is exposed to external mechanical shock. The damper 101 may be a foam substrate layer attached to the battery 230 by adhesive. A similar damper may alternatively (or in addition) be attached to any other components of the electronic module. A suitable adhesive for use in a medical device such as an inhaler to secure the foam substrate layer to the battery holder may be an acrylic adhesive, but any other suitable adhesive safe from a toxicology point of view may be used. The cap 110, battery 230, PCB 118, and/or heat stakes 212, 214 may be configured (shaped and/or positioned) within the electronic module 105 to position the foam substrate layer of damper 101 such that when the cap 110 of electronic module 105 is attached to the upper housing 140 of the inhaler, the foam substrate layer is loaded against a surface of the upper housing 140 i.e. the foam substrate layer is pushed against a surface of the upper housing 140 so as to compress the foam substrate. Although preferred, it is not essential for the foam substrate to be loaded against a surface of the upper housing 140 for the damper to be effective, i.e. there may be a gap between the foam substrate layer and a surface of the upper housing 140 and the gap may be less than 3 mm. A gap between the foam substrate and surface of the upper housing 140 may occur due to manufacturing tolerances.

When the inhaler 100 is dropped and the components of the electronic module 105 are exposed to a mechanical shock, the heat stakes 212, 214 attaching the PCB 118 to the cap 110 allow a degree of movement of the PCB 118 and the components attached thereto to mitigate against failure or any adverse effects that may occur from an impact. The movement of the PCB 118 occurs from elastic deformation of the heat stakes 212, 214 and/or from movement of the PCB 118 with respect to the heat stakes 212, 214.

The foam substrate layer of the damper 101 dampens energy transfer to and from the battery 230 by way of compressive damping (i.e. compression of the foam substrate layer against a surface of the upper housing 140) and prevents the battery 230 from vibrating excessively when the electronic module 105 is exposed to mechanical shock. The damper 101 reduces energy transferred to and/or from the battery 230, and accordingly reduces energy transfer to the heat stakes 212, 214,and the PCB 118, and thereby reduces the risk of the heat stakes 212, 214 failing or becoming damaged, the PCB 118 from entering into an erroneous state and draining the battery 230, and/or the tabs holding the PCB 118 or other parts of the device from peeling away from the battery 230.

The foam substrate layer of the damper 101 is disc shaped and smaller in size than the surface of the battery 230 it is attached to, the battery 230 being a coin cell battery with a diameter of 20 mm and the foam substrate layer having a diameter of 16 mm (i.e. 20% smaller in diameter). The foam substrate layer and may be positioned on the surface of the battery 230 so as to be offset from a central position thereof, as shown in FIG. 5. The foam substrate layer is offset on the surface of the battery 230 away from the heat stakes 212, 214 so that in the final stages of the manufacturing process of the electronics module 105, when the heat stakes 212, 214 are deformed/melted to fix the PCB 118 to the cap 110 the foam substrate layer does not thermally decompose.

The foam substrate layer of the damper 101 shown in FIGs 1 to 5 is 3.2 mm thick and is made from ethylene-vinyl acetate (EVA) closed cell foam with a 25% compression deflection of around 10 KPa. The foam substrate layer 101 is approximately 0.2 g which comprises 1.672 mcg/g acetaldehyde, 5.012 mcg/g methanol, 4.254 mcg/g 1-butanol and 57.882 mcg/g 2-ethylhexanol as volatile extractables.

Other suitable substrate layer with the desired dampening properties may be used, e.g. foams comprising polyurethane, silicone, neoprene, or polyethylene such as low-density polyethylene, so long as they are safe from a toxicology point of view. The foam substrate layer may be ISO 10993 compliant, for example MED 5634 Single-Coated Foam by Vancive^{™} Medical Technologies.

Gaskets, membranes (e.g. rubber membranes), filters and the like (not shown in the FIGs) may optionally be used to cover orifices 146 in order to avoid communication between the housing 190 and the PCB 118 and battery 230 in the electronic module 105, thereby preventing any medicament within housing 190 from communicating/interacting with electronic components of the electronic module 105. The means to prevent communication of the medicament with the electronic components of the module as described above are configured to allow the slider 116 to operate as described herein. Any means to prevent communication of the medicament with the electronic components of the module as described above may be configured so as to not inhibit the functionality of any sensors present in the electronic module 105.

The PCB 118 may include a sensor (not shown) that may provide information to a processor (not shown) about a patient's inhalation. The sensor may be a pressure sensor, such as a MEMS or NEMS pressure sensor (e.g., a barometric pressure sensor, a differential pressure sensor, etc.). The sensor may provide the information for example, using a pressure change and/or a pressure difference. The sensor may provide an instantaneous pressure reading to the processor and/or aggregated pressure readings over time. The processor may use the information to determine an air flow rate associated with the patient's inhalation through an air flow path in the inhaler. The processor may also use the information to determine the direction of air flow. That is, a negative change in air pressure through an air flow path within the inhaler may indicate that the patient has inhaled from the mouthpiece while a positive change in air pressure through the air flow path may indicate that the patient has exhaled into the mouthpiece.

The electronics module 105 may further include a wireless communication circuit, such as a Bluetooth chipset (e.g., a Bluetooth Low Energy chipset). As such, the electronics module 105 may provide a pressure measurement to an external device (e.g., a smartphone), which may perform additional calculations on the pressure measurement data, provide feedback to the user, and/or the like. The electronics module 105 may include a control circuit, which for example, may be part of the communication circuit.

Based on the information or signals received from the switch 222 and/or the sensor, the electronics module 105 may determine whether the mouthpiece cover 130 has been open or closed and whether a received pressure measurement exceeds a threshold or is within a specific pressure range, which may be indicative of whether the medication inhaled by a user has reached a predetermined or prescribed level. The pressure measurement threshold(s) and/or range(s) may be stored in a memory of the electronics module 105. When the predetermined threshold or range is met, the electronics module 105 may determine the state of the inhaler 100 and may generate a signal that indicates the state of the inhaler 100.

The electronics module 105 may include a memory (not shown) for storing data collected by the sensor (e.g., pressure measurements) and/or data generated by the processor (e.g., air flow rates). The stored data may be accessed by the processor and wirelessly communicated to an external device, such as a smartphone, via the wireless communication circuit. The memory may be non-removable memory and/or removable memory. The nonremovable memory may include random-access memory (RAM), read-only memory (ROM), a hard disk, or any other type of memory storage device. The removable memory may include a subscriber identity module (SIM) card, a memory stick, a secure digital (SD) memory card, and the like. The electronics module 105 may access information from, and store data in, a memory that is not physically located within the inhaler 100, such as on a server or a smartphone.

The processor of the electronics module 105 may comprise a microcontroller, a programmable logic device (PLD), a microprocessor, an application specific integrated circuit (ASIC), a field-programmable gate array (FPGA), or any suitable processing device, controller, or control circuit. The processor may comprise an internal memory.

The processor of the electronics module 105 may receive power from the battery 230, and may be configured to distribute and/or control the power to the other components in the electronics module 105. The battery 230 may be any suitable device for powering the electronics module 105. The battery 230 may be directly connected to one or more of the PCB, the sensor, the memory, and/or the transceiver of the electronics module 105.

The electronic modules and methods according to the disclosure are notable departures from the conventional electronic modules for use in medical devices and methods for manufacturing the same. The electronic modules disclosed herein provide improved robustness and resistance against damage or failure when dropped. Those skilled in the art will appreciate that the presently disclosed electronic modules and methods teach by way of example and not by limitation. Therefore, the matter contained in the above description or shown in the accompanying drawings should be interpreted as illustrative and not in a limiting sense. The following claims are intended to cover all generic and specific features described herein, as well as all statements of scope of the present electronic devices and method, which, as a matter of language, might be said to fall there between.

## Claims

1. An inhaler comprising an upper housing (140) and an electronic module (105), the electronic module comprising:
a cap (110) that is configured to be attached to the upper housing (140) of the inhaler,
a printed circuit board (118) attached to the cap (110) by one or more heat stakes (212, 214) that extend from a top, inner surface (220) of the cap (110),
a battery (230) connected to the printed circuit board (118), and
a damper (101) configured to dampen energy transfer to and/or from the battery (230) when the electronic module (105) is exposed to mechanical shock to reduce energy transfer to the heat stakes (212, 214) and the printed circuit board (118), wherein the damper (101) comprises a substrate which is positioned at a surface of said upper housing (140) of the inhaler thereby enabling the substrate to provide compressive damping against said surface.

2. The inhaler according to claim 1, wherein the battery (230) is mechanically and electronically connected to the printed circuit board by one or more connectors.

3. The inhaler of claim 2, wherein the one or more connectors are tabs (240, 242).

4. The inhaler of claims 2 or 3, wherein one part of each connector is attached to the battery (230) and another part of each connector is attached to the printed circuit board (118).

5. The inhaler according to any one of claims 2 to 4, wherein at least two connectors are attached to the battery (230) on different or opposed sides thereof.

6. The inhaler according to any preceding claim, wherein the substrate is a foam material.

7. The inhaler according to any preceding claim, wherein the substrate comprises rubber, polyurethane, silicone, neoprene, polyethylene-vinyl acetate, or polyethylene

8. The inhaler according to any preceding claim, wherein the substrate is from 0.1 to 10 mm thick, and/or wherein the substrate has a 25% compression force deflection of between 0.1 to 200 KPa.

9. The inhaler according to any preceding claim, wherein the damper (101) is attached to the battery (230).

10. The inhaler according to claim 9, wherein the substrate is adhesively applied to a surface of the battery (230).

11. The inhaler according to any preceding claim, wherein the battery connector(s) and/or the damper (101) are configured to position the substrate such that a distance between the substrate and said surface is less than 5 mm.

12. The inhaler according to claim 11, wherein the battery connector(s) and/or the damper (101) are configured to position the substrate to be loaded against the surface.

13. The inhaler according to any preceding claim, wherein the cap (110) is configured to be removably attached to the upper housing (130) of the inhaler.

## Patentansprüche

1. Inhalator, umfassend ein Gehäuseoberteil (140) und ein elektronisches Modul (105), wobei das elektronische Modul umfasst:
eine Kappe (110), die ausgestaltet ist, um an dem Gehäuseoberteil (140) des Inhalators befestigt zu werden, eine gedruckte Schaltplatine (118), die an der Kappe (110) mittels eines oder mehrerer Heißfügestege (212, 214) befestigt ist, die sich von einer oberen Innenoberfläche (220) der Kappe (110) erstrecken,
eine Batterie (230), die mit der gedruckten Schaltplatine (118) verbunden ist, und
einen Dämpfer (101), der ausgestaltet ist, um Energieübertragung zu und/oder von der Batterie (230) zu dämpfen, wenn das elektronische Modul (105) mechanischem Schock ausgesetzt ist, um Energieübertragung auf die Heißfügestege (212, 214) und die gedruckte Schaltplatine (118) zu reduzieren, wobei der Dämpfer (101) ein Substrat umfasst, das an einer Oberfläche des Gehäuseoberteils (140) des Inhalators positioniert ist, wodurch ermöglicht wird, dass das Substrat Kompressionsdämpfung gegen die Oberfläche bereitstellt.

2. Inhalator nach Anspruch 1, wobei die Batterie (230) mechanisch und elektronisch über einen oder mehrere Verbinder mit der gedruckten Schaltplatine verbunden ist.

3. Inhalator nach Anspruch 2, wobei der eine oder die mehreren Verbinder Laschen (240, 242) sind.

4. Inhalator nach den Ansprüchen 2 oder 3, wobei ein Teil jedes Verbinders an der Batterie (230) befestigt ist, und ein anderes Teil jedes Verbinders an der gedruckten Schaltplatine (118) befestigt ist.

5. Inhalator nach einem der Ansprüche 2 bis 4, wobei mindestens zwei Verbinder an der Batterie (230) an unterschiedlichen oder entgegengesetzten Seiten davon befestigt sind.

6. Inhalator nach einem der vorhergehenden Ansprüche, wobei das Substrat ein Schaummaterial ist.

7. Inhalator nach einem der vorhergehenden Ansprüche, wobei das Substrat Kautschuk, Polyurethan, Silikon, Neopren, Polyethylen-Vinylacetat oder Polyethylen umfasst.

8. Inhalator nach einem der vorhergehenden Ansprüche, wobei das Substrat 0,1 bis 10 mm dick ist, und/oder wobei das Substrat eine 25 % Kompressionskraftumlenkung zwischen 0,1 und 200 KPa hat.

9. Inhalator nach einem der vorhergehenden Ansprüche, wobei der Dämpfer (101) an der Batterie (230) befestigt ist.

10. Inhalator nach Anspruch 9, wobei das Substrat durch Klebung auf eine Oberfläche der Batterie (230) aufgebracht ist.

11. Inhalator nach einem der vorhergehenden Ansprüche, wobei der bzw. die Batterieverbinder und/oder der Dämpfer (101) ausgestaltet ist bzw. sind, um das Substrat so zu positionieren, dass ein Abstand zwischen dem Substrat und der Oberfläche kleiner als 5 mm ist.

12. Inhalator nach Anspruch 11, wobei der bzw. die Batterieverbinder und/oder der Dämpfer (101) ausgestaltet ist bzw. sind, um das Substrat so zu positionieren, dass es gegen die Oberfläche gespannt ist.

13. Inhalator nach einem der vorhergehenden Ansprüche, wobei die Kappe (110) ausgestaltet ist, um abnehmbar an dem Gehäuseoberteil (130) des Inhalators befestigt zu sein.

## Revendications

1. Inhalateur comprenant un boîtier supérieur (140) et un module électronique (105), le module électronique comprenant :
un capuchon (110) qui est conçu pour être fixé au boîtier supérieur (140) de l'inhalateur,
une carte de circuit imprimé (118) fixée au capuchon (110) par une ou plusieurs protubérances thermiques (212, 214) qui s'étendent à partir d'une surface supérieure interne (220) du capuchon (110),
une batterie (230) connectée à la carte de circuit imprimé (118), et
un amortisseur (101) conçu pour amortir un transfert d'énergie vers et/ou depuis la batterie (230) lorsque le module électronique (105) est exposé à un choc mécanique afin de réduire le transfert d'énergie vers les protubérances thermiques (212, 214) et la carte de circuit imprimé (118), dans lequel l'amortisseur (101) comprend un substrat qui est positionné au niveau d'une surface dudit boîtier supérieur (140) de l'inhalateur, ce qui permet au substrat d'assurer un amortissement compressif contre ladite surface.

2. Inhalateur selon la revendication 1, dans lequel la batterie (230) est reliée mécaniquement et électroniquement à la carte de circuit imprimé par un ou plusieurs connecteurs.

3. Inhalateur selon la revendication 2, dans lequel les un ou plusieurs connecteurs sont des languettes (240, 242) .

4. Inhalateur selon les revendications 2 ou 3, dans lequel une partie de chaque connecteur est fixée à la batterie (230) et une autre partie de chaque connecteur est fixée à la carte de circuit imprimé (118).

5. Inhalateur selon l'une quelconque des revendications 2 à 4, dans lequel au moins deux connecteurs sont fixés à la batterie (230) sur des côtés différents ou opposés de celle-ci.

6. Inhalateur selon une quelconque revendication précédente, dans lequel le substrat est un matériau alvéolaire.

7. Inhalateur selon une quelconque revendication précédente, dans lequel le substrat comprend du caoutchouc, du polyuréthane, de la silicone, du néoprène, du polyéthylène-acétate de vinyle ou du polyéthylène.

8. Inhalateur selon une quelconque revendication précédente, dans lequel le substrat a une épaisseur de 0,1 à 10 mm, et/ou dans lequel le substrat a une déflexion de force de compression de 25 % entre 0,1 et 200 KPa.

9. Inhalateur selon une quelconque revendication précédente, dans lequel l'amortisseur (101) est fixé à la batterie (230) .

10. Inhalateur selon la revendication 9, dans lequel le substrat est appliqué de manière adhésive à une surface de la batterie (230) .

11. Inhalateur selon une quelconque revendication précédente, dans lequel le ou les connecteurs de batterie et/ou l'amortisseur (101) sont conçus pour positionner le substrat de telle sorte qu'une distance entre le substrat et ladite surface soit inférieure à 5 mm.

12. Inhalateur selon la revendication 11, dans lequel le ou les connecteurs de batterie et/ou l'amortisseur (101) sont conçus pour positionner le substrat à charger contre la surface.

13. Inhalateur selon une quelconque revendication précédente, dans lequel le capuchon (110) est conçu pour être fixé de manière amovible au boîtier supérieur (130) de l'inhalateur.
